# EUROPÄISCHE PATENTSCHRIFT

(11) **EP 2 088 988 B1**
(45) Veröffentlichungstag und Bekanntmachung des Hinweises auf die Patenterteilung: **01.06.2016**
(21) Anmeldenummer: 07846553.1
(22) Anmeldetag: 09.11.2007
(51) Int. Cl.: A61K 8/31, A61K 8/34, A61K 8/37, A61Q 17/00, A61Q 19/00

(54) **HAUTSCHUTZMITTEL, INSBESONDERE KÄLTESCHUTZCREME**
SKIN PROTECTIVE MEDIUM, IN PARTICULAR COLD PROTECTIVE CREAM
AGENT DE PROTECTION DE LA PEAU, EN PARTICULIER CRÈME DE PROTECTION CONTRE LE FROID

(30) Priorität: 10.11.2006 DE 102006053360
(43) Veröffentlichungstag der Anmeldung: 19.08.2009
(73) Patentinhaber: Deb IP Limited, Denby, Derbyshire DE5 8JZ (GB)
(72) Erfinder: VEEGER, Marcel, 47574 Goch (DE); ALLEF, Petra, 47807 Krefeld (DE)
(74) Vertreter: Elsy, David
(86) Internationale Anmeldenummer: PCT/EP2007/009726
(87) Internationale Veröffentlichungsnummer: WO 2008/055692

(56) Entgegenhaltungen:
- EP-A- 1 371 355
- WO-A-85/04101
- WO-A-99/65598
- WO-A-03/082239
- WO-A-2004/105712
- WO-A-2005/054222
- WO-A-2006/018104
- DE-A1- 4 345 186
- US-A1- 2003 235 539
- US-A1- 2004 047 828

## Beschreibung

Die Erfindung betrifft ein silikonfreies Hautschutzmittel, insbesondere ein Mittel zum Schutz der Haut bei extremer Kälte.

Die menschliche Haut ist mit einer Oberfläche von ca. 1,5 bis 2,0 m² das oberflächengrößte Organ des Menschen, das für den Körper lebenswichtige Funktionen wahrnimmt. Hierfür enthält die Haut Blut- und Lymphgefäße, durch deren Wände hindurch der Austausch von Lymphflüssigkeit, Gasen, Nähr- und Abfallstoffen stattfinden kann, um z.B. Ernährung und Stoffwechsel zu gewährleisten. Weitere Funktionen der Haut sind die Regelung der Körpertemperatur, der Schutz des Körpers gegen Austrocknung und gegen äußere mechanische, chemische und bakterielle Einwirkungen. So halten die Sekrete von Hauttalgdrüsen die Haut geschmeidig und helfen bei der Regulierung des Wasserhaushaltes der Haut. Darüberhinaus vermittelt die Haut unter anderem über freie Nervenendigungen dem Organismus Tast-, Wärme- und Kälte- und Schmerzreize und hat somit die Funktion eines Sinnesorgans.

Die mannigfaltigen für Hygiene, Körperpflege und Kosmetik angebotenen Produkte, wie z.B. Hautreinigungs-, Hautschutz- und Hautpflegeprodukte dienen daher nicht nur primär der Reinigung, dem Schutz bzw. der Pflege der Haut, sondern sie erhalten auch die Lebensqualität und sichern das Wohlbefinden des Menschen.

Als Körperhülle stellt die menschliche Haut die Verbindung, aber zugleich auch Abgrenzung des menschlichen Körpers mit seiner Außenwelt dar. Insbesondere erfolgt über sie die Kontaktaufnahme mit allem, was der menschliche Organismus zum Leben benötigt - aber auch was sie gefährden kann.

Hautschutzmittel sollen die menschliche Haut vor den unterschiedlichsten Gefährdungen der Außenwelt schützen, wie z.B. Witterungseinflüsse, Wasser und wässrige Lösungen, Chemikalien sowie Verschmutzungen jedweder Art. Üblicherweise überziehen solche Hautschutzmittel in wasserfreier Form die menschliche Haut mit einem "Barriere"- bzw. Schutzfilm, der von der Haut nicht absorbiert werden kann. Idealerweise ist dieser Schutzfilm dergestalt auf der Haut appliziert, daß sein Vorhandensein von der Umwelt nicht bemerkt wird, da er für diese unsichtbar bzw. für den Anwender auf dessen Haut nicht spürbar ist. In diesem Zusammenhang sind Hautschutzpräparate zu nennen, die als "unsichtbarer Handschuh" im Handel erhältlich sind und die als völlig wasserundurchlässige Barrierepräparate beim Arbeiten mit ätzenden oder wässrigen toxischen Lösungen Verwendung finden bzw. Schutz vor organischen Lösungsmitteln bieten sollen.

Nachteilig an solchen Präparaten ist jedoch, daß die natürliche Wasserdampfabgabe über die Haut beeinträchtigt wird. Aufgrund des damit verbundenen Wärme- und Feuchtigkeitsstaus wird die Akzeptanz für die Anwendung solcher Produkte durch Beschäftigte, die täglich vielfach mit wässrigen und/oder organischen Hautschadstoffen in Berührung kommen, herabgesetzt.

Im Handel werden Hautschutzmittel in großer Zahl in den unterschiedlichsten Zubereitungsformen z.B. in Form von Stiften, Salben, Cremes, Gelees, als W/O-, Misch- und O/W-Emulsionen sowie als alkoholische Lotionen angeboten, die dann je nach Verwendungszweck und Rezeptur als Sportöle bzw. -cremes, Allwettercremes, Babycremes, Hautschutzsalben, Hautschutzöle aber auch Fettstifte, Sonnenschutzmittel etc. bezeichnet werden, wobei Hautsalben, Hautcremes, Hautlotionen, Hautöle und Hautgele die wichtigsten Typen darstellen. Übliche Barrieremittel in diesen Hautschutzpräparaten sind vor allem Paraffin-Kohlenwasserstoffe wie mineralische Öle, Vaseline etc. aber auch mineralische und pflanzliche Wachse einschließlich Siliconöle und Silikonwachse. Hautcremes und Hautlotionen basieren vor allem auf Emulsionen vom O/W-(Öl-in-Wasser) oder W/O- Typ (Wasser-in-Öl). Hauptbestandteile der Öl-Phase (auch Fett- oder Lipidphase) können dann Fettalkohole, Fettsäuren, Fettsäureester, Wachse, Vaseline, Paraffine aber auch sonstige Fette und Öle hauptsächlich natürlichen Ursprungs sein. Neben den für Hautschutzprodukte üblicherweise verwendeten Emulsionen vom O/W- oder W/O- Typ sind auch multiple Emulsionen zur Herstellung von kosmetischen und pharmazeutischen Produkten bekannt. Bei solchen multiplen Emulsionen handelt es sich um Emulsionen von Emulsionen, deren wichtigste Vertreter multiple Wasser/Ol/Wasser (W/O/W)- sowie Öl/Wasser/Öl (O/W/O)- Emulsionen vielfach in der Patentliteratur beschrieben sind.

Die wässrige Phase kann u.a. wasserlösliche Pflegewirkstoffe, die feuchtigkeitsregulierend bzw. feuchtigkeitsbewahrend sind, enthalten.

Welche der vorgenannten Emulsionstypen in einem Hautschutzprodukt Anwendung finden, richtet sich vornehmlich danach, welcher Schutzzweck mit dem Produkt verfolgt werden soll bzw. gegen welche Arbeitsstoffe das Produkt schützen soll.

Zum Schutz vor Witterungseinflüssen, z.B. extremer Kälte u.a. in Verbindung mit star-ken Winden, wie sie beispielsweise in den Kälte- und Wüstenregionen der nördlichen und südlichen Hemisphäre, aber auch in den hochalpinen Regionen der Erde anzutreffen sind, werden wasserfreie Hautschutzpräparate verwendet. Hierbei handelt es sich üblicherweise um Fettcremes mit einem hohen Anteil an Paraffinen und Fettalkoholen, die die Haut gegen Feuchtigkeits- und Wärmeverluste schützen. Für diese Anwendung macht man sich den vorgenannten Effekt zunutze, daß der applizierte Schutzfilm der natürlichen Wasserdampfabgabe, d.h. der physiologischen Hautatmung und damit verbunden der Temperaturregulierung der Haut entgegenwirkt.

Diesen extremen Witterungsverhältnissen, die in den Kälte- und Wüstenregionen der Erde vorherrschen, müssen auch die Hautschutzpräparate Rechnung tragen, die für den Einsatz in diese Regionen bestimmt sind. So müssen solche Präparate nicht nur der dort, insbesondere im Winter vorherrschenden extremen Kälte widerstehen können, sondern in den kurzen Sommern werden auch Temperaturen bis 40°C und mehr erreicht. In manchen ariden Klimazonen der Erde betragen die Temperaturen > +40°C am Tage und sinken in der Nacht auf unter 0°C. Diese klimatischen Bedingungen stellen hohe Anforderungen an die Stabilität und damit verbunden die Transport- und Lagerfähigkeit solcher Produkte. Dies gilt umso mehr als auch zu berücksichtigen ist, daß ein "Einfrieren" der Produkte nicht ausgeschlossen werden kann bzw. es ist während der Gebrauchsdauer solcher Hautschutzpräparate von mehreren Zyklen auszugehen, in denen die Produkte gefroren sind und anschließend wieder aufgetaut werden.

Diesen rigiden klimatischen Bedingungen halten viele derzeit im Markt befindliche Hautschutzpräparate nur unzureichend stand, insbesondere im Hinblick auf deren "Gefrier-Tau-Belastbarkeit". Auch frieren die meisten Produkte bereits bei -5°C komplett ein. Da die kosmetischen bzw. pharmazeutischen Zubereitungen solcher Produkte bei diesen tiefen Temperaturen nicht mehr aus Tuben entnehmbar sind, werden sie in Form von Dosen, Tiegel etc. angeboten. Die Anwendung solcher Präparate ist mühsam, insbesondere da sich das gefrorene Produkt nur schlecht auf der Haut verteilen läßt. Darüberhinaus ist die Applikation aus einer Tube in hygienischer Hinsicht der Entnahme aus einem Tiegel vorzuziehen.

Es ist bekannt, daß man vorstehende Problematik mittels Hautschutzmitteln auf Basis von Silikonen begegnen kann. So ist beschrieben, daß Silikone, wie z.B. Silikonöle und Silikonwachse hervorragende Barrieremittel sind, die in Hautschutzmitteln eine besondere Stabilität aufweisen. Sie sind wärmebeständig und gegen die Einwirkung von ätzenden Chemikalien außerordentlich beständig. Darüberhinaus sind solche silikonhaltigen Präparate stark hydrophob und hautungefährlich, weil sie physiologisch verträglich, d.h. nicht gesundheitsschädlich, sondern vielmehr auch hautverträglich sind. Aufgrund der geringen Oberflächenspannung von Silikonölen lassen sie sich leicht auf der Haut verteilen. Vorteilhaft ist auch, daß bei Silikonschichten auf der Haut im Gegensatz zu Paraffinen, Vaseline etc. keine Gefahr von Wärmestauungen auf der Haut besteht.

Nachteilig an solchen silikonhaltigen Präparaten ist jedoch, daß diese Präparate auf Gegenständen z.B. auf Werkstoffen bzw. Werkstücken Rückstände hinterlassen können, wenn solche Werkstücke per Hand einem weiteren Arbeitsgang zugeführt werden. So können z.B. die Beschäftigten bei zu lackierenden Werkstücken solche silikonhaltigen Hautschutzmittel nicht einsetzen, da diese sehr schwer entfernbaren Silikonrückstände bei der Weiterverarbeitung dieser Werkstücke wie z. B. Lackieren oder Vulkanisieren stark störend wirken, die insbesondere zu Lackierungsfehlern wie z.B. Lackbenetzungsstörungen bzw. Kraterbildung auf den Werkstücken führen. Daher können, insbesondere in der Automobil- und Lackindustrie sowie der gummiverarbeitenden Industrie silikonhaltige Hautschutzprodukte trotz ihrer hervorragenden Schutzwirkung und Akzeptanz nicht zum Einsatz kommen.

Es besteht somit ein großes Bedürfnis nach silikonölfreien Hautschutzmitteln, die nicht nur eine gute Wirksamkeit beim Schutz gegenüber Witterungseinflüssen, Wasser und wässrige Lösungen, Chemikalien sowie Verschmutzungen zeigen, sondern die auch ein Eigenschaftsprofil haben, daß den in den Kälte- und Wüstenregionen der Erde vorherrschenden Bedingungen, insbesondere im Hinblick auf Transport- und Lagerfähigkeit und auf "Gefrier-Tau-Belastbarkeit" hinreichend Rechnung trägt.

Aufgabe der vorliegenden Erfindung war es daher, solche silikonfreien Hautschutzmittel zu entwickeln, die vorzugsweise in Form einer Kälte-Hautschutzcreme bei sogenannten "Out-Door-Workem" in den Kälteregionen der nördlichen und südlichen Hemisphäre, aber auch in kalten/ hautaustrocknenden Wüstenregionen der Erde einsetzbar sind, insbesondere sollen die Mittel bei mindestens -18°C noch aus einer Tube applizierbar, d.h. pastös bzw. fließ- und/oder streichfähig sein und darüberhinaus soll die Stabilität über einen Zeitraum von wenigstens 3 Monaten bei +40° bzw. 1 Monat bei +50°C zu gewährleistet sein sowie eine "Gefrier-Tau-Belastbarkeit" von wenigstens 3 Gefrier-Tau-Zyklen aufweisen.

Diese Aufgabe wurde überraschend durch ein silikonfreies Hautschutzmittel, gemäß Anspruch 1.

In einer bevorzugten Ausführungsform weisen die erfindungsgemäßen Hautschutzmittel eine Viskositätsdifferenz im Temperaturintervall von +4°C bis +50°C von höchster und niedrigster Viskosität des Hautschutzmittels einen Wert im Bereich von 0 bis ≤ 15000 mPas und besonders bevorzugt von 0 bis ≤ 5000 mPas auf.

Erfindungsgemäß können als Komponente a.) jedes üblicherweise für kosmetische und/oder pharmazeutische Formulierungen einsetzbare Öl, das die Haut mit einem Schutz- oder Barrierefilm überzieht, insbesondere die bereits oben genannten Paraffin-Kohlenwasserstoffe wie mineralische Öle, beispielsweise Vaseline etc. einschließlich pflanzlicher und tierischer Öle, soweit diese nicht wie z.B. die silikonhaltigen Präparate zu unerwünschten Rückständen auf Werkstoffen und Werkstücken führen, entweder allein oder als Mischung mit einem weiteren oder mehreren anderen Ölen verwendet werden, wenn das Öl bzw. das Olgemisch einen Pourpoint gemäß DIN ISO 30 16 von ≤ - 10°C aufweist.

Bevorzugt einzusetzende Öle der Komponente a.) sind Kohlenwasserstoffe und Carbonate wie z.B.
Arlamol® HD (isohexadecan) der Firma Uniqema, Gouda, Holland, Pourpoint von - 70°C,
Paraffinum perliquidum, Pourpoint von -15°C oder
Tegosoft® DEC (Diethylhexyl Carbonat) der Fa. Goldschmidt GmbH, Essen, Deutschland, Pourpoint von -30°C.

Darüberhinaus können auch andere für kosmetische und/oder pharmazeutische Formulierungen geeignete Öle als Komponente a.) eingesetzt werden, wenn sie Bestandteil einer Ölmischung sind, die die zwingende Bedingung, wonach das Ölgemisch einen Pourpoint gemäß DIN ISO 30 16 von ≤ - 10°C aufweisen muß, erfüllt ist.

Exemplarisch sei hier Tegosoft® OS (Ethylhexylstearate), Schmelzpunkt von ca. 8°C genannt.

Vorzugsweise sind die einzusetzenden Öle der Komponente a.) insbesondere 10 bis 50 Gew.-%, bezogen auf die Gesamtzusammensetzung des Hautschutzmittels, vorzugsweise 10 bis 40 Gew.-% und besonders bevorzugt 15 bis 30 Gew.-% in den erfindungsgemäßen Hautschutzmitteln enthalten.

Erfindungswesentlich ist, daß die Öle der Komponente a.) in Kombination mit wenigstens einem Polyol der Komponente b.) in den erfindungsgemäßen Hautschutzmitteln enthalten sind. Es zeigte sich, daß die erfindungsgemäßen Hautschutzmittel, insbesondere in Form einer Kälteschutzcreme bzw. W/O-Emulsion, die bei mindestens-18°C noch aus einer Tube applizierbar, d.h. pastös bzw. fließ- und/oder streichfähig sein und darüberhinaus eine Stabilität über einen Zeitraum von wenigstens 3 Monaten bei +40° bzw. 1 Monat bei +50°C gewährleisten sowie eine "Gefrier-Tau-Belastbarkeit" von wenigstens 3 Gefrier-Tau-Zyklen aufweisen sollen, durch die Verwendung von Ölen bzw. Ölgemischen mit einem Pourpoint gemäß DIN ISO 30 16 von ≤ - 10°C allein nicht erhalten werden.

Als Polyole der Komponente b.), die allein oder als Mischung mit einem weiteren oder mehreren anderen Polyolen eingesetzt werden können, kommen übliche für kosmetische und/oder pharmazeutische Formulierungen geeignete, d.h. physiologisch verträgliche Polyalkohole bzw. Polyhydoxyverbindungen, vorzugsweise mit 2 bis 15 Kohlenstoffatomen und mindestens 2 Hydroxylgruppen in betracht. Exemplarisch sind hierzu nennen mehrwertige Alkohole wie geradkettige, verzweigte oder cyclische Alkanole mit 2 bis 15, vorzugsweise 2 bis 6 Kohlenstoffatomen, wobei Glyzerin und/oder 1,2 Propandiol besonders bevorzugt sind. Darüberhinaus können Glykole, wie z.B. Polyethylenglykole, Polypropylenglykole aber auch Zucker und Zuckerderivate, vorzugsweise Fructose, Glucose, Saccharose, Zuckeralkohole, insbesondere Sorbit, Mannit etc. in den erfindungsgemäßen Hautschutzmitteln enthalten sein. Der Anteil der Komponente b.) in den Hautschutzmitteln beträgt hierbei mindestens 7,5 bis 40 Gew.-%, bezogen auf die Gesamtzusammensetzung des Hautschutzmittels, vorzugsweise 10 bis 35 und besonders bevorzugt 15 bis 25 Gew.-%.

Weiterhin enthalten die erfindungsgemäßen Hautschutzmittel 1 bis 10 Gew.-%, vor-zugsweise 2 bis 8, besonders bevorzugt 2 bis 4 Gew.-%, bezogen auf die Gesamtzusammensetzung des Hautschutzmittels, mindestens einen Emulgator, der allein oder als Mischung mit einem weiteren oder mehreren anderen Emulgatoren enthalten sein kann.

Erfindungsgemäß bevorzugt für die erfindungsgemäßen Hautschutzmittel als Emulgatoren sind Ester der Polyhydroxystearinsäure und/oder der Polyricinolsäure. Besonders bevorzugt sind Polyglycerinpartialester von Polyhydroxystearinsäure und mehrfunktionellen Carbonsäuren, die erhältlich sind durch Veresterung
a.) eines Polyglyceringemisches mit
b.) Polyhydroxystearinsäure und
c.) Di- und/oder Tricarbonsäuren und gegebenenfalls/oder mit
d.) Dimerfettsäuren, und
e.) Fettsäuren mit 6 bis 22 C-Atomen,
deren Herstellung in der DE 103 33 443 A1 beschrieben wird.

Erfindungsgemäß besonders bevorzugte Emulgatoren der Komponente c.) sind Polyglycerinpartialester der Polyhydroxystearinsäure, die von der Firma Goldschmidt GmbH, Essen unter der Bezeichnung ISOLAN® GPS (Polyglyceryl- 4- Diisostearate Polyhydroxystearate Sebacate) erhältlich sind. Weitere im Handel erhältliche Emulgatoren, insbesondere auf Basis von Polyhydroxystearinsäure und/oder Polyricinolsäure sind Crester® PR (Polyglycerl-3 ricinoleate) von der Firma Croda, Dehymuls® PGPH (Polyglyceryl-2 Dipolyhydroxystearate) von der Firma Cognis sowie Arlacel® P135 (PEG-30 Dipolyhydroxystearate)oder Arlacel® 1689V (Polyglyceryl-3 Polyricinoleate) von der Firma Uniqema, die erfindungsgemäß im Bereich von 1 bis 10 Gew.-%, bevorzugt 2 bis 4 Gew.-%, bezogen auf die Gesamtzusammensetzung des Hautschutzmittels, die entweder allein oder als Emulgatormischung in den erfindungsgemäßen Produkten enthalten sein können.

Darüberhinaus können der Emulgatormischung weitere Coemulgatoren zugefügt werden. Insbesondere kann der Emulgatormischung zusätzlich mindestens ein Alkyl-und/oder Alkylenglucosid und einem Fettalkohol und/oder Partialglyceriden und gege-benenfalls weitere Coemulgatoren enthalten. Solche Emulgatoren werden in der DE 196 12 084 A1 beschrieben.

Optional enthalten die erfindungsgemäßen Hautschutzmittel als Komponente d.) bis höchstens 2 Gew.-%, vorzugsweise bis 0,25 bis 1 Gew.-% mindestens ein Wachs, das entweder allein oder als Mischung mit einem weiteren oder mehreren anderen Wachsen enthalten sein kann, wie beispielsweise pflanzliche und/oder tierische Wachse, insbesondere Candelillawachs, Carnaubawachs, Japanwachs, Espartowachs, Korkwachs, Reiskeimölwachs, Guaramawachs, Ouricurywachs, Montanwachs, Jojobawachs, Sheabutter, Beerenwachs, Bienenwachs, Schellackwachs, Walrat, Lanolin, Bürzelfett, Mineralwachse, wie z.B. Ceresin oder Ozokerit (Erdwachs), petrochemische Wachse, insbesondere Petrolatum, Paraffinwachse sowie mikrokristalline Wachse. Darüberhinaus können auch chemisch modifizierte Wachse und synthetische Wachse als Komponente d.) in den erfindungsgemäßen Hautschutzmitteln enthalten sein, insbesondere auch Montanesterwachse, Sasolwachse, hydrierte Jojobawachse, Polyalkylenwachse, Polyethylenglycolwachse, aber auch chemisch modifizierte Fette, wie z.B. hydrierte Pflanzenöl, insbesondere hydriertes Ricinusöl, hydrierte Cocosfettglyceride, Triglyceride etc. Besonders bevorzugt enthalten die erfindungsgemäß Hautschutzmittel hydriertes Ricinusöl, das beispielsweise unter der Handelsmarke CUTI-NA® HR von der Firma Cognis vertrieben wird, bzw. Mineralwachse wie z.B. Ceresin, das als mikrokristallines Wachs unter der Handelsbezeichnung Paracera® W 80 erhalten werden kann.

Neben Wasser können die erfindungsgemäßen silikonfreien Hautschutzmittel gegebenenfalls, Hilfs-, Zusatz- und/oder Wirkstoffe wie z.B. Farbstoffe, Lösungsvermittler, Komplexbildner, Sequestrierungsmittel, Lichtschutzfilter oder Parfüm- bzw. Duftstoffe, pH-Regulatoren, Stabilisatoren, vorzugsweise Cetearylalkohol und/oder hydrierte Ricinusöle, wie z.B. Trihydroxystearin, Konservierungsmittel, Antioxidantien und/oder ölige oder wässrige Pflegekomponenten als weitere Komponenten enthalten, insbesondere in den für kosmetische und/oder pharmazeutische Formulierungen üblichen Mengen von vorzugsweise 0,05 bis 30 Gew.-%, bezogen auf das Gesamtgewicht des Hautschutzmittels, wobei der Fachmann den Gewichtsanteil dieser Komponenten so wählt, daß es zu keiner Beeinträchtigung der Barrierebildung der erfindungsgemäßen Hautschutzmittel aufgrund möglicher penetrationsfördernden Eigenschaften dieser Hilfs-, Zusatz- und/oder Wirkstoffe kommt.

Erfindungsgemäß bevorzugt sind silikonfreie Hautschutzmittel, insbesondere Kälteschutzmittel, in Form von W/O-Emulsionen, die die Komponenten, jeweils bezogen auf die Gesamtzusammensetzung des Hautschutzmittels,
a.) mindestens 10 bis 50 Gew.-% mindestens eines Öls mit einem Pourpoint gemäß DIN ISO 30 16 von ≤ - 10°C,
b.) mindestens 10 bis 40 Gew.-% mindestens eines Polyols,
c.) mindestens 1 bis 10 Gew.-% mindestens eines Emulgators,
d.) optional bis höchstens 2 Gew.-% mindestens eines Wachses,
enthalten, mit der Maßgabe, daß sich die Mengen mit Wasser und gegebenenfalls weiteren Zusatzstoffen zu 100 Gew.-% ergänzen.

Die erfindungsgemäßen Hautschutzmittel können besonders vorteilhaft zum Schutz vor extremen klimatischen Witterungseinflüssen, wie sie in den Kälte- und Wüstenregionen der nördlichen und südlichen Hemisphäre, aber auch in den hochalpinen Regionen der Erde angetroffen werden, verwendet werden. So wurde überraschenderweise gefunden, daß die erfindungsgemäßen Formulierungen sehr gut der Hautaustrocknung, die in diesen Klimaten vorherrscht, entgegen wirkt, obwohl diese Formulierungen einen Ölgehalt aufweisen, der im Vergleich zu handelsüblichen Produkten wie beispielsweise Kälteschutzcremes eher gering ist. Weiterhin ist dem Fachmann bekannt, daß Polyole allein im allgemeinen im kalten Klima der Haut Wasser entziehen, so daß deren Einsatz in Kälteschutzformulierungen eigentlich als nicht zielführend zu betrachten ist, weil deren Verwendung in Kälteschutzmitteln dem gewünschten Anforderungsprofil entgegen zu stehen scheint.

Darüberhinaus bieten die erfindungsgemäßen Produkte nicht nur, wie bereits ausgeführt, Schutz vor den in diesen Klimaten verherrschenden Witterungsverhältnissen, insbesondere extreme Kälte u.a. in Verbindung mit starken Winden, und sie weisen auch eine verbesserte Transport- und Lagerfähigkeit einschließlich verbesserter "Gefrier-Tau-Belastbarkeit" auf, sondern sie sind auch noch bei mindestens -18°C aus einer Tube applizierbar, d.h. pastös bzw. fließ- und streichfähig und lassen sich selbst bei diesen Temperaturen auf der Haut verteilen. So weisen die erfindungsgemäßen Produkte bei T= -18°C Viskositäten von ca. 20.000 mPas auf bzw. besonders vorteilhaft sind die Viskositäten bei einer Temperatur von -24°C noch kleiner 50.000 mPas, insbesondere zeigt der Viskositätsverlauf bei sinkender Temperatur - bestimmt nach der Methode in G.Schramm; "Einführung in die praktische Viskosimetrie"; 1981, Gebrüder Haake GmbH, 5. Auflage (siehe Experimenteller Teil) - bei d(y)/dt = 10s⁻¹, daß ein Viskositätsmaximum von maximal 600 Pas im Temperaturintervall keinesfalls überschritten wird.

Besonders vorteilhaft ist insbesondere auch, daß sie über einen Zeitraum von wenigstens 3 Monaten bei +40° bzw. wenigstens 1 Monat bei +50°C stabil sind und eine "Gefrier-Tau-Belastbarkeit" von wenigstens 3 Gefrier-Tau-Zyklen zeigen.

Aufgrund dieses Eigenschaftsprofils sind die erfindungsgemäßen Hautschutzmittel in besonderer Weise für sogenannte "Out-Door-Worker" geeignet, wie z.B. Beschäftigte in der Erdöl- und Erdgasexploration, insbesondere auf Erdölplattformen beispielsweise in der Nordsee sowie in der Bergbauindustrie, in den Kälteregionen der nördlichen und südlichen Hemisphäre, aber auch in den kalten/ hautaustrocknenden Wüstenregionen der Erde. Exemplarisch sind hierfür die rohstoffreichen Gebiete Alaskas, Sibiriens bzw. Zentralasiens zu nennen. Besonders bevorzugt können die erfindungsgemäßen Hautschutzmittel auch im gewerblichen Bereich bei längeren Arbeiten in Kühl-/ Gefrierlagerhäusern angewendet werden.

Weiterhin vorteilhaft ist, daß die erfindungsgemäßen Hautschutzmittel, insbesondere Schutz vor wässrigen Noxen bieten, so daß sie als Schutzmittel beispielsweise für Arbeitsplätze wie z.B. in der metallverarbeitenden, gummiverarbeitenden Industrie Anwendung finden können, da eine Beeinträchtigung von Arbeitsprozessen durch Rückstände von Silikonverbindungen z.B. auf Werkstoffen bzw. Werkstücken ausgeschlossen werden kann.

In der Land- und Forstwirtschaft, aber auch bei entsprechenden Freizeit- und Hobbyaktivitäten wie z.B. im Winter- und Bergsport können die erfindungsgemäßen Hautschutzmittel, insbesondere bei extremen Witterungsverhältnissen beispielsweise als sogenannte Allwettercremes, Sportöle bzw. -cremes etc. ebenfalls verwendet werden.

Im folgenden wird die Erfindung anhand von Beispielen erläutert. Diese Erläuterungen sind lediglich beispielhaft und schränken die hier vorliegende Erfindung nicht ein. Die angegebenen Mengenangaben, Anteile und Prozentanteile sind, soweit nicht anders angegeben, auf das Gewicht und die Gesamtmenge bzw. auf das Gesamtgewicht der erfindungsgemäßen Hautschutzmittel bezogen.

### Experimenteller Teil:

### 1. Pourpointbestimmung:

Die Pourpointbestimmung für die in den erfindungsgemäßen Hautschutzmitteln enthaltenden Öle der Komponente a.) erfolgte nach DIN ISO 3016 Oktober 1982.

Gemäß dieser Norm wird der Pourpoint als diejenige Temperatur, d.h. niedrigste Temperatur definiert, bei welcher ein Öl bzw. Ölgemisch eben noch fließt.

Hierzu wird entsprechend den in der DIN ISO 3016 festgelegten Bedingungen eine Ölprobe unter vorangehender Aufwärmung kontrolliert abgekühlt wird, wobei in Temperaturabständen von je 3°C die Ölprobe auf ihre Fließfähigkeit geprüft wird, bis die Temperatur des Pourpoints für die jeweilige Probe erreicht ist.

### 2. Viskositätsbestimmung:

Die für die erfindungsgemäßen Hautschutzmittel ermittelten Viskositätswerte wurden mit einem Viskosimeter der Fa. Brookfield Engineering Inc., nämlich einem Brookfield RVT Rotationsviskosimeter mit Spindelset 1-7 erhalten.

Die Herstellung der erfindungsgemäßen Hautschutzmittel, erfolgt mittels üblicherweise bekannter Vorrichtungen bzw. Verfahren, wobei die Hautschutzmittel vorzugsweise als cremige Mittel oder als fließfähige viskose Pasten erhalten werden, insbesondere besonders bevorzugt als W/O-Emulsionen.

Erfindungsgemäß bevorzugte Hautschutzmittel, insbesondere Kälteschutzcremes mit Ausnahme der Vergleichsbeispiele D, N, R, H, I und J haben beispielsweise folgende Zusammensetzungen:

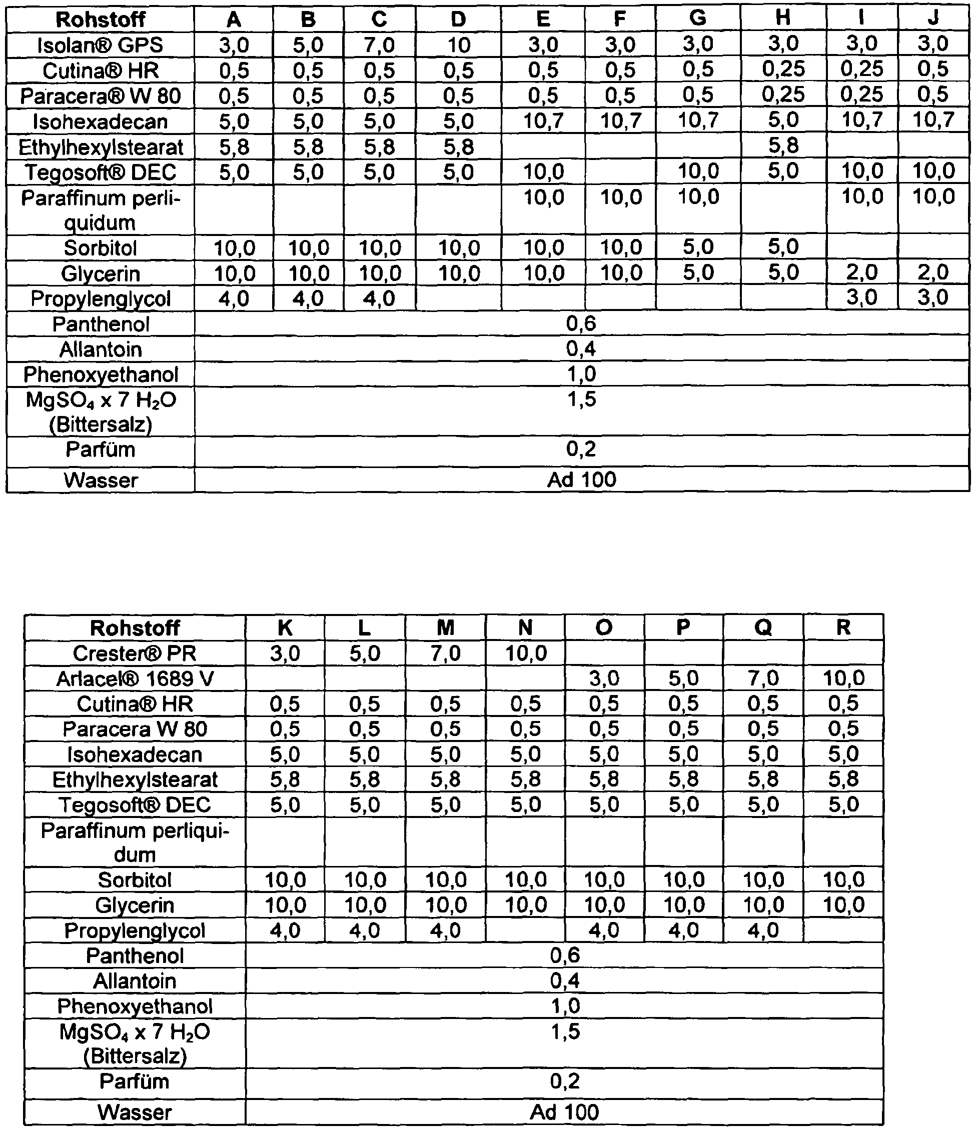

Es konnte experimentell gezeigt werden, daß die Verwendung von niedrig siedenden Ölen wie z.B. in Beispiel I allein nicht zu den erfindungsgemäßen Formulierungen mit dem gewünschten Anforderungsprofil führt, wonach die Mittel bei mindestens -18°C noch aus einer Tube applizierbar, d.h. pastös bzw. fließ- und/oder streichfähig sein müssen und die Stabilität über einen Zeitraum von wenigstens 3 Monaten bei +40° bzw. 1 Monat bei +50°C gewährleistet ist bei einer "Gefrier-Tau-Belastbarkeit" von wenigstens 3 Gefrier-Tau-Zyklen. So war die Formulierung gemäß Beispiel I bei Raumtemperatur wasserdünn und war schließlich bei 40°C instabil. Bei -20°C war die Formulierung komplett gefroren und nicht mehr streichfähig.

Auch ein Anheben des Wachsgehaltes entsprechend Beispiel J führte nicht zu Produkten mit dem vorgenannten Anforderungsprofil. Bei -20°C war die Formulierung ebenfalls komplett gefroren und nicht mehr streichfähig.

Gleiches gilt, wenn man entsprechend Beispiel H den Ölgehalt absenkt, so erhält man zwar bei Raumtemperatur eine glatte Creme, die auch bei hohen Temperaturen stabil bleibt, jedoch friert diese Formulierung bereits bei unter -5°C ein.

Die Formulierung gemäß dem erfindungsgemäßen Beispiel E hat bei 4°C eine Viskosität von 3.000 mPas und bei 50°C eine Viskosität von 2.500 mPas bzw. bei -18°C weist sie eine Viskosität von 40.000 mPas auf (gemessen mit Brookfield RVT Rotationsviskosimeter mit Spindelset 1-7).

### 3. Bestimmung der Applizierbarkeit, d.h. der Pastösität bzw. Fließ- und/oder Streichfähigkeit der erfindungsgemäßen Hautschutzmittel

Die Applizierbarkeit der erfindungsgemäßen Hautschutzmittel bzw. der Vergleichsprodukte wurde entsprechend G.Schramm; "Einführung in die praktische Viskosimetrie"; 1981, Gebrüder Haake GmbH, 5. Auflage bestimmt.

Hiernach werden beim Ausdrücken einer Creme aus einer Tube mit einem Öffnungsdurchmesser von 5mm unter Annahme einer konstanten, laminaren Fließgeschwindigkeit von Q = 1 (cm/s) eine Geschwindigkeitsgefälle d(y)/dt von 81 (s⁻¹) erreicht - bei einem Öffnungsdurchmesser von 4 mm bereits 159 (s⁻¹). Dies entspricht der Formel: D =4/π * Q/R³
D= Geschwindigkeitsgefälle d(γ)/dt
Q= Fließgeschwindigkeit cm/s
R= Tubenöffnungsradius

Die Schubspannung T ist hierbei eine von der Viskosiät q der Substanz abhängige Größe. Sie wird definiert als T = Kraft/Fläche = η * D. Bei einem konstanten Geschwindigkeitsgefälle ist T direkt proportional η.

Erfindungsgemäß werden hier die Ausdrückbarkeit eines Hautschutzmittels aus einer Tube mit Applizierbarkeit, d.h. Pastösizität bzw. Fließ- und/oder Streichfähigkeit gleichgesetzt. Diese Methode wurde gewählt, weil hiermit die Ausdrückbarkeit eines fließfähigen Mediums aus einer Tube bestimmbar ist, damit das erfindungsgemäße Kriterium, wonach die erfindungsgemäßen Mittel bei mindestens -18°C, vorzugsweise bei mindestens -20°C noch aus einer Tube applizierbar, d.h. pastös bzw. fließ- und/oder streichfähig sein müssen, verifiziert und damit auch gewährleistet werden kann.

Gemäß dieser Methode werden beim Einreiben einer Handcreme Geschwindigkeitsgefälle zwischen D = 10000 - 20000 (S⁻¹) erreicht.
Die Messungen von diversen Formulierungen bei diskreten Temperaturen zwischen +20°C und -30°C wurden mit einem Oszillationsrheometer Physica MCR 301 von Anton Paar durchgeführt.

In der nachfolgenden Tabelle bzw. dem hierzu zugehörigen Kurvendiagramm sind beispielhaft die Messungen, von erfindungsgemäßen und nicht erfindungsgemäßen Formulierungen bzw. eines Handelsproduktes, die bei einem konstanten Geschwindigkeitsgefälle von d(y)/dt = 10s⁻¹ durchgeführt wurden, dargestellt :

**Viskositätsverlauf in [Pas]**

| Temperatur | Beispiel A | Beispiel E | Handelsware | Beispiel H | W/O Lotion |
|---|---|---|---|---|---|
| 1 | 8,94 | 8,96 | 134 | 13,6 | 14 |
| -1 | 9,15 | 9,11 | 137 | 13,7 | 14,3 |
| -2 | 9,26 | 9,28 | 151 | 13,9 | 14,6 |
| -3 | 9,42 | 9,44 | 178 | 14,3 | 14.9 |
| -4 | 9,7 | 9,62 | 185 | 15 | 15,3 |
| -5 | 9,7 | 9,81 | 211 | 15,3 | 15,6 |
| -6 | 9,51 | 9,99 | 224 | 15,6 | 16 |
| -7 | 10,1 | 10,2 | 257 | 15,8 | 16,4 |
| -8 | 10,5 | 10,4 | 294 | 16,1 | 16,8 |
| -9 | 10,8 | 10,6 | 309 | 16,4 | 17,2 |
| -10 | 11 | 10,8 | 337 | 16,7 | 17,7 |
| -11 | 11,4 | 11,1 | 376 | 17 | 18,2 |
| -12 | 12,8 | 11,3 | 411 | 17,4 | 24,5 |
| -13 | 14,7 | 11,7 | 440 | 19,5 | 40,2 |
| -14 | 16,4 | 12 | 397 | 21,9 | 52,7 |
| -15 | 18 | 12,4 | 384 | 24,1 | 63,6 |
| -16 | 19,8 | 12,8 | 403 | 30,1 | 73,9 |
| -17 | 21,1 | 13,2 | 443 | 276 | 84,3 |
| -18 | 23,6 | 13,6 | 453 | 549.000 | 382 |
| -19 | 25,3 | 14,1 | 496 | 1.640* | 67.000.000 |
| -20 | 27,6 | 14,6 | 1.050 | 456* | 341.000.000 |
| -21 | 29,6 | 15,2 | 920 | 173* | 1.340.000.000 |
| -22 | 31,9 | 15,7 | 1.050 | 109* | 1.740.000.000 |
| -23 | 34,7 | 16,4 | 1.360 | 85,5* | 1.840.000.000 |
| -24 | 44,8 | 17,1 | 2.020 | 74,5* | 959.000.000 |
| -25 | 132 | 17,8 | 16.700 | 76* | -11.300.000.000 |
| -26 | 364 | 18,6 | 34.900 | 79,2* | 2.270.000.000 |
| -27 | 532 | 19,5 | 102.000 | 84,6* | 7.010.000.000 |
| -28 | 487 | 20,9 | 446.000 | 89,8* | 4.020.000.000 |
| -29 | 471 | 22 | 1.900.000 | 89,9* | 3.560.000.000 |
| -30 | 421 | 23,4 | 7.550.000 | 97,5* | 1.710.000.000 |

| | | | | | |
|---|---|---|---|---|---|
| *Produkt war bei dieser Temperatur instabil | | | | | |

Der Tabelle bzw. dem diesbezüglichen Kurvendiagramm ist zu entnehmen, daß die erfindungsgemäßen Formulierungen bei T= -18°C Viskositäten von ca. 20.000 mPas aufweisen bzw. besonders vorteilhaft bei einer Temperatur von -24°C noch kleiner 50.000 mPas sind. Darüberhinaus liegt das Viskositätsmaximum bei den erfindungsgemäßen Formulierungen bei max. 600 Pas im Temperaturbereich bis - 30°C.

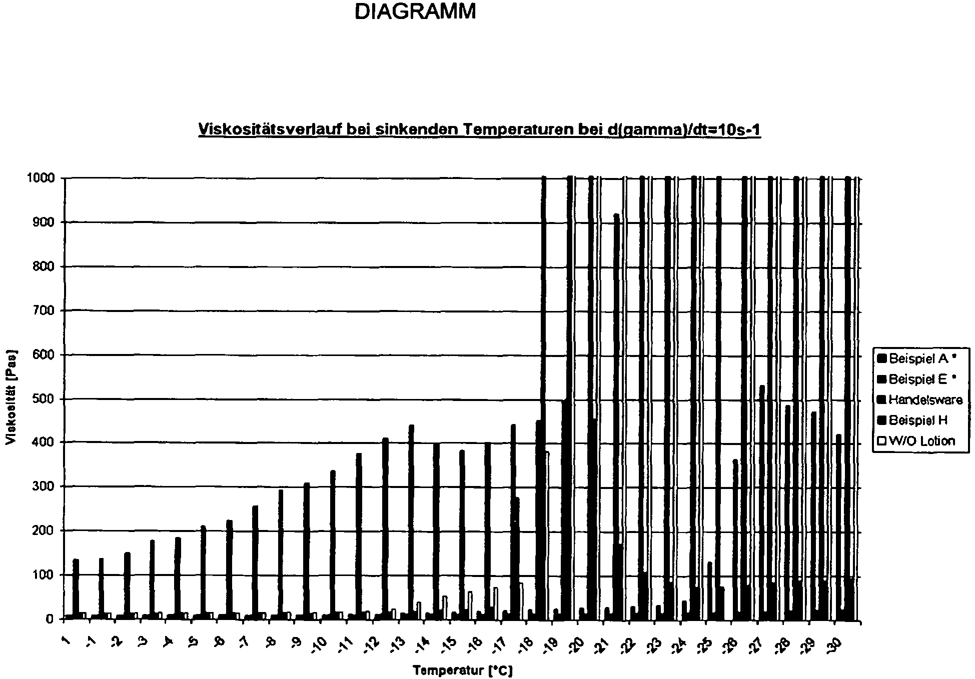

## Patentansprüche

1. Silikonfreies Hautschutzmittel, insbesondere Kälteschutzmittel enthaltend die Komponenten
a.) 10 bis 50 Gew.-% mindestens eines Öls mit einem Pourpoint gemäß DIN ISO 30 16 von ≤ - 10°C, ausgewählt aus Kohlenwasserstoffen und Carbonaten,
b.) 15 bis 40 Gew.-% mindestens eines Polyols, ausgewählt aus Glycerin, 1,2-Propandiol, Polyethylenglykol, Polypropylenglykol, Fructose, Glucose, Saccharose, Sorbit und Mannit,
c.) 1 bis 8 Gew.-% mindestens eines Emulgators,
d.) optional mindestens ein Wachs,
**dadurch gekennzeichnet, daß** die Viskositätsdifferenz im Temperaturintervall von +4°C bis +50°C von höchster und niedrigster Viskosität des Hautschutzmittels einen Wert im Bereich von 0 bis ≤ 20000 mPas aufweist.

2. Hautschutzmittel nach Anspruch 1, **dadurch gekennzeichnet, daß** die Viskositätsdifferenz im Temperaturintervall von +4°C bis + 50°C von höchster und niedrigster Viskosität des Hautschutzmittels einen Wert im Bereich von 0 bis ≤ 15000 m Pas aufweist.

3. Hautschutzmittel nach Anspruch 1 oder 2, **dadurch gekennzeichnet, daß** die Viskositätsdifferenz, im Temperaturintervall von +4°C bis +50°C von höchster und niedrigster Viskosität des Hautschutzmittels einen Wert im Bereich von 0 bis ≤ 5000 mPas aufweist.

4. Hautschutzmittel nach einem der Ansprüche 1 bis 3, **dadurch gekennzeichnet, daß** der Anteil des Wachses oder des Wachsgemisches höchstens 2 Gew.-%, bezogen auf die Gesamtzusammensetzung des Hautschutzmittels, beträgt.

5. Hautschutzmittel nach einem der Ansprüche 1 bis 4, **dadurch gekennzeichnet, daß** das Hautschutzmittel eine W/O-Emulsion ist, die die Komponenten, jeweils bezogen auf die Gesamtzusammensetzung des Hautschutzmittels,
a.) mindestens 10 bis 50 Gew.-% mindestens eines Öls mit einem Pourpoint gemäß DIN ISO 30 16 von ≤ - 10°C,
b.) mindestens 15 bis 40 Gew.-% mindestens eines Polyols,
c.) mindestens 1 bis 8 Gew.-% mindestens eines Emulgators,
d.) optional bis höchstens 2 Gew.-% mindestens eines Wachses,
enthält, mit der Maßgabe, daß sich die Mengen mit Wasser und gegebenenfalls weiteren Zusatzstoffen zu 100 Gew.-% ergänzen.

6. Hautschutzmittel nach einem der Ansprüche 1 bis 5, **dadurch gekennzeichnet, daß** die Komponente c.) mindestens ein Polyhydroxystearinsäurederivat und/oder mindestens ein Polyricinolsäurederivat oder eine Mischung hiervon ist.

7. Hautschutzmittel nach Ansprüch 6, **dadurch gekennzeichnet, daß** das Polyhydroxystearinsäurederivat mindestens ein Polyglycerinpartialester von Polyhydroxystearinsäure ist, insbesondere Polyglyceryl-4-Diisostearate Polyhydroxystearate Sebacate und/oder Polyglyceryl-2 Dipolyhydroxystearate oder eine Mischung hiervon ist.

8. Hautschutzmittel nach den Ansprüchen 6 oder 7, **dadurch gekennzeichnet, daß** der Emulgator oder die Emulgatormischung der Komponente c.) aus der Gruppe Polyglyceryl-4-Diisostearate Polyhydroxystearate Sebacate, Polyglyceryl-3 ricinoleate, Polyglyceryl-2 Dipolyhydroxystearate, PEG-30 Dipolyhydroxystearate, Polyglyceryl-3 Polyricinoleate ausgewählt ist.

9. Verwendung der Hautschutzmittel nach einem der Ansprüche 1 bis 8 als Kälteschutzcreme.

## Claims

1. Silicone-free skin protection agent, in particular cold protection agent, containing the following components:
a) 10 to 50 % by weight of at least one oil having a pour point according to DIN ISO 3016 of ≤ -10°C, said oil being selected from hydrocarbons and carbonates,
b) 15 to 40 % by weight of at least one polyol, selected from glycerine, propane-1,2-diol, polyethylene glycol, polypropylene glycol, fructose, glucose, saccharose, sorbite and mannitol,
c) 1 to 8 % by weight of at least one emulsifier,
d) optionally at least one wax,
**characterized in that** the difference in viscosity, in the +4°C to +50°C temperature range, between the highest and the lowest viscosity of the skin protection agent has a value ranging between 0 and ≤ 20,000 mPas.

2. Skin protection agent as claimed in claim 1, **characterized in that** the difference in viscosity, in the +4°C to +50°C temperature range, between the highest and the lowest viscosity of the skin protection agent has a value ranging between 0 and 15,000 mPas.

3. Skin protection agent as claimed in claims 1 or 2, **characterized in that** the difference in viscosity, in the +4°C to + 50°C temperature range, between the highest and lowest viscosity of the skin protection agent has a value ranging between 0 and ≤ 5,000 mPas.

4. Skin protection agent as claimed in any one of claims 1 to 3, **characterized in that** the proportion of said wax or wax mixture amounts to no more than 2 % by weight based on the total composition of the skin protection agent.

5. Skin protection agent as claimed in any one of claims 1 to 4, **characterized in that** said skin protection agent is a water-in-oil emulsion which comprises the following components, each based on the total composition of the skin protection agent:
a) at least 10 to 50 % by weight of at least one oil having a pour point according to DIN ISO 3016 of ≤ -10°C,
b) at least 15 to 40 % by weight of at least one polyol,
c) at least 1 to 8 % by weight of at least one emulsifier,
d) optionally no more than 2 % by weight of at least one wax,
with the proviso that these quantities, after adding water and other additives as necessary, add up to 100 % by weight.

6. Skin protection agent as claimed in any one of claims 1 to 5, **characterized in that** component c) is at least one polyhydroxystearic acid derivative and/or at least one polyricinol acid derivative, or a mixture thereof.

7. Skin protection agent as claimed in claim 6, **characterized in that** said polyhydroxystearic acid derivative is at least one polyglycerol partial ester of polyhydroxystearic acid, in particular polyglyceryl-4 diisostearate, polyhydroxystearate sebacate and/or polyglyceryl-2 dipolyhydroxystearate, or a mixture thereof.

8. Skin protection agent as claimed in any one of claims 6 or 7, **characterized in that** said emulsifier or emulsifier mixture of component c) is selected from the group consisting of polyglyceryl-4 diisostearate, polyhydroxystearate sebacate, polyglyceryl-3 ricinoleate, polyglyceryl-2 dipolyhydroxystearate, PEG-30 dipolyhydroxystearate, polyglyceryl-3 polyricinoleate.

9. Use of the skin protection agents as claimed in any one of claims 1 to 8 as a cold protection cream.

## Revendications

1. Agent de protection de la peau sans silicone, en particulier agent de protection contre le froid, comprenant les composants suivants :
a) 10 à 50 % en poids d'au moins une huile ayant un point d'écoulement d'après la norme DIN ISO 30 16 des ≤ -10 °C, choisie parmi les hydrocarbures et les carbonates,
b) 15 à 40 % en poids d'au moins un polyol, choisi parmi la glycérine, le 1,2-propanediol, le polyéthylène glycol, le polypropylène glycol, le fructose, le glucose, le saccharose, le sorbitol et le mannitol,
c) 1 à 8 % en poids d'au moins un émulsifiant,
d) éventuellement au moins une cire,
**caractérisé en ce que** la différence de viscosité dans l'intervalle de températures allant de +4 °C à +50 °C de viscosité maximale et minimale de l'agent de protection de la peau présente une valeur située dans la plage allant de 0 à ≤ 20000 mPas.

2. Agent de protection de la peau selon la revendication 1, **caractérisé en ce que** la différence de viscosité dans l'intervalle de températures allant de +4 °C à +50 °C de viscosité maximale et minimale de l'agent de protection de la peau présente une valeur située dans la plage allant de 0 à ≤ 15000 mPas.

3. Agent de protection de la peau selon la revendication 1 ou 2, **caractérisé en ce que** la différence de viscosité dans l'intervalle de températures allant de +4 °C à +50 °C de viscosité maximale et minimale de l'agent de protection de la peau présente une valeur située dans la plage allant de 0 à ≤ 5000 mPas.

4. Agent de protection de la peau selon l'une des revendications 1 à 3, **caractérisé en ce que** la proportion de cire ou du mélange de cires atteint au maximum 2 % en poids par rapport à la composition totale de l'agent de protection de la peau.

5. Agent de protection de la peau selon l'une des revendications 1 à 4, **caractérisé en ce que** l'agent de protection de la peau est une émulsion E/H, qui comprend les composants suivants, respectivement par rapport à la composition totale de l'agent de protection de la peau,
a) au moins 10 à 50 % en poids d'au moins une huile ayant un point d'écoulement d'après la norme DIN ISO 30 16 de ≤ -10 °C,
b) au moins 15 à 40 % en poids d'au moins un polyol,
c) au moins 1 à 8 % en poids d'au moins un émulsifiant,
d) éventuellement jusqu'à 2 % en poids au maximum d'au moins une cire,
à condition que les quantités constituent 100 % en poids en complétant avec de l'eau et éventuellement d'autres additifs.

6. Agent de protection de la peau selon l'une des revendications 1 à 5, **caractérisé en ce que** le composant c) est au moins un dérivé d'acide polyhydroxystéarique et/ou au moins un dérivé d'acide polyricinolique ou un mélange de ceux-ci.

7. Agent de protection de la peau selon la revendication 6, **caractérisé en ce que** le dérivé d'acide polyhydroxystéarique est au moins un ester partiel de polyglycérine d'acide polyhydroxystéarique, en particulier le sébacate de polyhydroxystéarate diisostéarate de polyglycéryle-4 et/ou le dipolyhydroxystéarate de polyglycéryle-2 ou un mélange de ceux-ci.

8. Agent de protection de la peau selon les revendications 6 ou 7, **caractérisé en ce que** l'émulsifiant ou le mélange d'émulsifiants du composant c) est choisi dans le groupe constitué du sébacate de polyhydroxystéarate diisostéarate de polyglycéryle-4, du ricinoléate de polyglycéryle-3, du dipolyhydroxystéarate de polyglycéryle-2, du dipolyhydroxystéarate de PEG-30, du polyricinoléate de polyglycéryle-3.

9. Utilisation des agents de protection de la peau selon l'une des revendications 1 à 8 comme crème de protection contre le froid.
